# FASCICULE DE BREVET EUROPEEN

(11) **EP 3 170 466 B1**
(45) Date de publication et mention de la délivrance du brevet: **21.06.2023**
(21) Numéro de dépôt: 16202035.8
(22) Date de dépôt: 02.10.2009
(51) Int. Cl.: A61B 17/80

(54) **IMPLANT ORTHOPEDIQUE SOUS FORME D'UNE PLAQUE DESTINEE A ETRE FIXEE ENTRE DEUX PARTIES D'OS**
ORTHOPÄDISCHES IMPLANTAT IN FORM EINER PLATTE ZUR FIXIERUNG ZWISCHEN ZWEI KNOCHENTEILEN
ORTHOPEDIC IMPLANT IN THE FORM OF A PLATE TO BE FIXED BETWEEN TWO BONE PARTS

(30) Priorité: 02.10.2008 FR 0856694
(43) Date de publication de la demande: 24.05.2017
(62) Demande divisionnaire de: 09756159.1
(73) Titulaire: Stryker European Operations Holdings LLC, Kalamazoo, MI 49002 (US)
(72) Inventeur: PRANDI, Bernard, 35700 Rennes (FR); WAPNER, Keith, Philadelphia, 19103 (US); WAPNER, Peter W, Media, 19063 (US); WAPNER, Charles P, Media, 19063 (US)
(74) Mandataire: Regimbeau

(56) Documents cités:
- EP-A1- 1 897 509
- WO-A1-95/16403
- WO-A1-02/098306
- DE-U1- 8 227 727
- US-A1- 2008 114 360

## Description

L'invention se rattache au secteur technique des implants orthopédiques.

Plus particulièrement, l'invention concerne une plaque pour arthrodèse ou ostéosynthèse destinée à être fixée entre deux parties d'os.

D'une manière parfaitement connue pour un homme du métier, ce type de plaque comprend, généralement, des trous pour l'engagement de vis permettant de réaliser une arthrodèse entre deux os ou une ostéosynthèse entre deux fragments osseux. C'est le cas, par exemple, pour les os de la main ou du pied, sans pour cela exclure d'autres applications, notamment dans le domaine du rachis. En fonction du cas pathologique à traiter, ces plaques peuvent être de forme générale rectiligne ou présenter d'autres formes géométriques. Un exemple de plaque pour arthrodèse ou ostéosynthèse destinée à être fixée entre deux parties d'os est décrit notamment dans le document EP 1 897 509 A1.

A partir de cet état de la technique, l'un des problèmes que se propose de résoudre l'invention est d'améliorer, d'une manière sure et efficace, la compression entre les parties d'os assujetties à la plaque et selon une direction précise.

Pour résoudre le problème posé d'améliorer la compression entre les deux parties d'os considérées, selon l'invention, la plaque présente au moins un agencement apte à permettre de positionner au moins une vis d'une manière inclinée par rapport au plan défini par ladite plaque selon un angle compris entre 30° et 60° environ.

Selon une forme avantageuse de réalisation, l'agencement est constitué par une zone inclinée selon l'angle compris entre 30° et 60°, et présentant un trou pour l'engagement de la vis. La zone inclinée résulte d'une découpe et d'une déformation d'une partie de la plaque.

Dans une autre forme de réalisation, l'agencement est constitué par un trou incliné selon l'angle compris entre 30° et 60° pour l'engagement de la vis.

Compte tenu du problème posé à résoudre, l'agencement est situé sur une partie déterminée de la longueur de la plaque pour que la vis assure la mise en compression des deux parties d'os.

L'invention est exposée ci-après plus en détail à l'aide des figures des dessins annexés dans lesquels :
- la figure 1 est une vue en perspective d'une forme de réalisation de la plaque ;
- la figure 2 est une vue de profil de la plaque ;
- les figures 3 et 4 sont des vues en perspective montrant le montage de la plaque entre deux parties d'os et la composition de ces dernières, au moyen de la plaque selon l'invention, les parties d'os étant présentées d'une manière schématique.

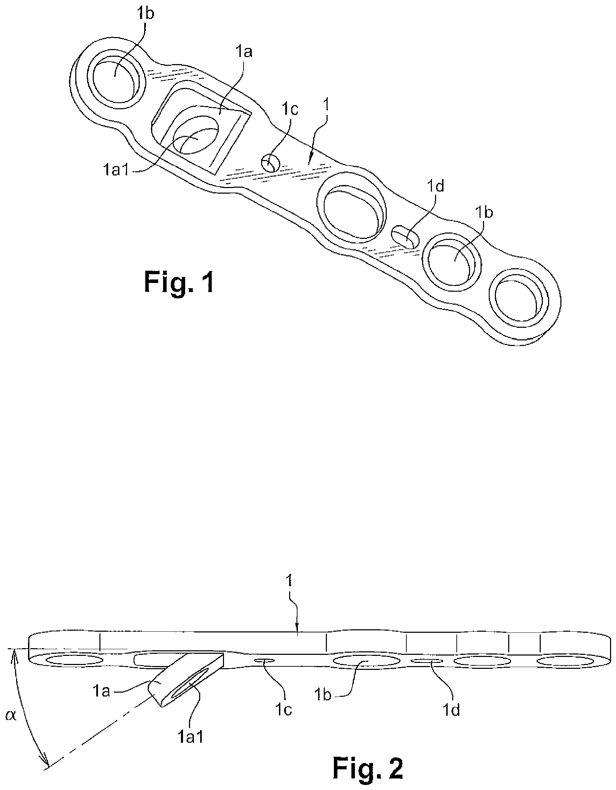

Selon l'invention, la plaque (1) présente au moins un agencement (la) apte à permettre de positionner au moins une vis (2), d'une manière inclinée, selon un angle α compris entre 30° et 60° par rapport au plan défini par ladite plaque (figure 2)

Dans une forme de réalisation, l'agencement (la) est constitué par une zone inclinée qui résulte d'une découpe et d'une déformation d'une partie de la plaque. Par exemple, la déformation est consécutive à une opération de découpage - poinçonnage. Cette zone inclinée constitue une nervure qui présente un trou (1a1) pour l'engagement de la vis (2). La nervure inclinée (la) est formée sur une partie déterminée de la longueur de la plaque pour qu'après engagement, la vis (2) assure la mise en compression des deux parties d'os, comme il sera indiqué dans la suite de la description.

Dans une autre forme de réalisation, pour permettre une orientation angulaire de la vis (2), selon un angle compris entre 30° et 60° environ, l'agencement (la) peut être constitué par un trou incliné. A noter que la nervure (la) permet une adaptation de l'angle en fonction du cas pathologique à traiter étant donné qu'il est possible de déformer à volonté cette nervure. Autrement dit, l'angle peut être réglé directement par le chirurgien sur quelques degrés en bloc opératoire avec un instrument adapté.

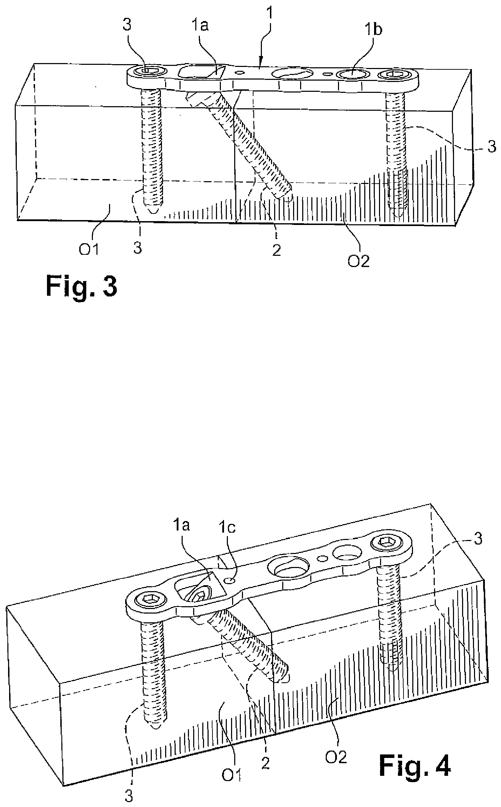

On renvoie aux figures 3 et 4 qui montrent le positionnement de la plaque (1) entre deux parties d'os (O1) et (O2) :
- Après réalisation des ostéotomies, un gabarit de la plaque, qui ne présente pas de nervures, permet de déterminer la position de cette nervure.
- Après avoir déterminé le positionnement de la nervure, le chirurgien réalise un logement correspondant, avec une râpe adaptée.
- Après positionnement de la plaque présentant la nervure, le chirurgien met en place une ou deux vis (3), d'un côté du foyer d'ostéosynthèse de l'arthrodèse considéré du côté de la nervure. On peut positionner, éventuellement, une broche de fixation temporaire dans un plot adapté.
- La vis (2) est ensuite engagée dans le trou (1a1) de la nervure (la) pour mettre le foyer de fracture en compression.
- Une fois la compression effectuée, le chirurgien peut visser une ou plusieurs autres vis (3) de fixation complémentaire et retirer la broche temporaire de maintien

D'une manière connue, cette plaque (1) présente des trous lisses et/ou taraudés (1b) pour l'engagement des vis de fixation (3) vissées dans les parties d'os (O1) et (O2), comme il ressort des figures 3 et 4.

De même, la plaque (1) peut présenter au moins un logement (le) pour l'introduction d'une broche en vue d'assurer une fixation temporaire de ladite plaque (1). Avantageusement, la plaque (1) peut présenter un logement (lc) pour l'introduction d'une broche du côté de l'une des parties de l'os (O1) et un autre logement (ld) pour l'introduction d'une autre broche du côté de l'autre partie d'os (O2).

Compte tenu de l'effet de compression recherchée, telle qu'indiquée précédemment, le logement (le) est constitué par un trou circulaire dont le diamètre correspond sensiblement à celui de la broche (4), tandis que l'autre logement (1d) peut être constitué par une lumière oblongue.

Ces dispositions permettent donc à l'os de coulisser sous la plaque (1) au moment du vissage, tout en assurant une compression selon une direction précise, généralement suivant l'axe de la plaque. Les broches sont de tout type connu et approprié, et parfaitement connu pour un homme du métier.

La plaque (1) peut présenter différentes formes géométriques, de sorte que les trous (la) notamment peuvent être alignés ou être disposés, en totalité ou en partie, selon les sommets d'un triangle ou d'un quadrilatère. Ces dispositions, en triangle ou en quadrilatère des vis, améliorent la stabilité du montage.

A noter également que la plaque (1), quelle que soit sa forme géométrique, peut être cintrée longitudinalement, pour s'adapter à la courbure de l'os permettant, en conséquence, aux vis (2) de former un angle entre elles.

Les avantages ressortent bien de la description.

## Revendications

1. Système incluant un implant ayant une structure sous forme d'une plaque (1) pour comprimer l'un contre l'autre une première (O1) et une deuxième (O2) partie d'os séparées par une jointure, l'implant comprenant :
la plaque (1) ayant une surface supérieure, une surface inférieure de contact osseux, et une pluralité de trous (1b) formés à travers les surfaces supérieure et inférieure, un premier trou de la pluralité de trous (1b) étant positionnable d'un premier côté de la jointure, et un deuxième trou de la pluralité de trous (1b) étant positionnable d'un deuxième côté de la jointure, les premier et deuxième trous étant adaptés pour recevoir des premier et deuxième éléments de fixation (3) respectivement, un axe central du premier trou s'étendant dans la première (O1) partie d'os mais pas dans la deuxième (O2) partie d'os, et un axe central du deuxième trou s'étendant dans la deuxième (O2) partie d'os mais pas dans la première (O1) partie d'os ; et
un agencement (la) constitué par une nervure formant un trou incliné (lal) s'étendant à travers la plaque (1) selon un angle compris entre 30° et 60° par rapport à la surface inférieure de contact osseux, le trou incliné (1a1) étant formé à une position sur une partie déterminée d'une longueur de la plaque (1) et adapté pour recevoir un troisième élément de fixation (2), un axe central du trou incliné (1a1) étant incliné par rapport à un axe longitudinal de la plaque de sorte que lorsque le troisième élément de fixation (2) est inséré à travers le trou incliné (1a1), il est agencé pour s'étendre dans la première (O1) partie d'os, à travers la jointure, et dans la deuxième (O2) partie d'os, et pour comprimer l'un contre l'autre la première (O1) et la deuxième (O2) partie d'os selon l'axe longitudinal de la plaque,
**caractérisé en ce que** le système comprend en outre un gabarit de la plaque à utiliser pour déterminer le positionnement de l'agencement (la) contre l'os, le gabarit n'incluant pas de nervure.

2. Système selon la revendication 1, dans lequel les premier et deuxième trous sont des trous de verrouillage.

3. Système selon la revendication 2, dans lequel les premier et deuxième trous comprennent un filetage pour venir en prise avec les premier et deuxième éléments de fixation (3).

4. Système selon l'une quelconque des revendications 1 à 3, dans lequel la plaque (1) est incurvée pour s'adapter à la courbure des première (O1) et deuxième (O2) parties d'os, la courbure de la plaque permettant à au moins deux éléments de fixation de former un angle l'un avec l'autre.

5. Système selon l'une quelconque des revendications 1 à 4, dans lequel au moins trois trous de la pluralité de trous (1b) sont agencés aux sommets d'un triangle.

6. Système selon l'une quelconque des revendications 1 à 4, dans lequel au moins quatre trous de la pluralité de trous (1b) sont agencés aux angles d'un quadrilatère.

7. Système selon l'une quelconque des revendications 1 à 4, dans lequel le trou incliné (1a1) est aligné avec le premier trou et avec le deuxième trou de la pluralité de trous (1b).

8. Système selon l'une quelconque des revendications 1 à 7, dans lequel l'agencement (la) est situé entre et s'étend sous des côtés adjacents de la plaque, de sorte que l'agencement est recevable dans une cavité formée dans au moins une parmi les première (O1) et deuxième (O2) parties d'os.

9. Système selon l'une quelconque des revendications 1 à 8, dans lequel l'agencement s'étend vers le bas par rapport à la surface inférieure et est situé par conséquent sous un logement formé dans la plaque (1), le logement est délimité par des parois latérales s'étendant à travers les surfaces supérieure et inférieure de la plaque, les parois latérales étant dimensionnées pour permettre une insertion du troisième élément de fixation (2) à travers ledit logement et dans le trou incliné (1a1).

10. Système selon la revendication 9, dans lequel le trou incliné (1a1) a un premier diamètre, et une tête du troisième élément de fixation (2) a un deuxième diamètre, le deuxième diamètre étant plus grand que le premier diamètre.

11. Système selon l'une quelconque des revendications 1 à 10, comprenant en outre des vis destinées à être insérées dans la pluralité de trous de l'implant.

## Patentansprüche

1. System, das ein Implantat mit einer Struktur in Form einer Platte (1) einschließt, um einen ersten (01) und einen zweiten (02), durch eine Verbindungsstelle getrennten Knochenabschnitt aneinanderzudrücken, wobei das Implantat umfasst:
die Platte (1) mit einer oberen Oberfläche, einer unteren Knochenkontakt-Oberfläche und einer Vielzahl von durch die obere und untere Oberfläche ausgebildeten Löchern (1b), wobei ein erstes Loch von der Vielzahl von Löchern (1b) auf einer ersten Seite der Verbindungsstelle positionierbar ist und ein zweites Loch von der Vielzahl von Löchern (1b) auf einer zweiten Seite der Verbindungsstelle positionierbar ist, wobei das erste und das zweite Loch jeweils zur Aufnahme von einem ersten und einem zweiten Befestigungselement (3) geeignet sind, wobei sich eine zentrale Achse des ersten Lochs im ersten (01) Knochenabschnitt, aber nicht im zweiten (02) Knochenabschnitt erstreckt, und sich eine zentrale Achse des zweiten Lochs im zweiten (02) Knochenabschnitt, aber nicht im ersten (01) Knochenabschnitt erstreckt; und
eine Anordnung (1a), gebildet von einem Steg, der ein geneigtes Loch (1a1) bildet, der sich durch die Platte (1) in einem Winkel erstreckt, der zwischen 30° und 60° im Verhältnis zur unteren Knochenkontakt-Oberfläche liegt, wobei das geneigte Loch (1a1) in einer Position auf einem bestimmten Abschnitt einer Länge der Platte (1) gebildet und zur Aufnahme eines dritten Befestigungselements (2) geeignet ist, wobei eine zentrale Achse des geneigten Lochs (1a1) im Verhältnis zu einer Längsachse der Platte geneigt ist, so dass, wenn das dritte Befestigungselement (2) durch das geneigte Loch (1a1) eingesetzt ist, es angeordnet ist, um sich in den ersten (01) Knochenabschnitt durch die Verbindungsstelle und in den zweiten (02) Knochenabschnitt zu erstrecken und um den ersten (01) und den zweiten (02) Knochenabschnitt gemäß der Längsachse der Platte aneinanderzudrücken;
**dadurch gekennzeichnet, dass** das System ferner eine Schablone der Platte umfasst, die zu verwenden ist, um die Positionierung der Anordnung (1a) am Knochen zu bestimmen, wobei die Schablone keinen Steg einschließt.

2. System nach Anspruch 1, wobei das erste und das zweite Loch Arretierlöcher sind.

3. System nach Anspruch 2, wobei das erste und das zweite Loch ein Gewinde umfassen, das mit dem ersten und dem zweiten Befestigungselement (3) in Eingriff kommt.

4. System nach einem der Ansprüche 1 bis 3, wobei die Platte (1) gekrümmt ist, um sich an die Krümmung des ersten (01) und zweiten (02) Knochenabschnitts anzupassen, wobei die Krümmung der Platte mindestens zwei Befestigungselementen erlaubt, einen Winkel miteinander zu bilden.

5. System nach einem der Ansprüche 1 bis 4, wobei mindestens drei Löcher von der Vielzahl von Löchern (1b) an den Spitzen eines Dreiecks eingerichtet sind.

6. System nach einem der Ansprüche 1 bis 4, wobei mindestens vier Löcher von der Vielzahl von Löchern (1b) an den Ecken eines Vierecks eingerichtet sind.

7. System nach einem der Ansprüche 1 bis 4, wobei das geneigte Loch (1a1) mit dem ersten Loch und mit dem zweiten Loch von der Vielzahl von Löchern (1b) aufgereiht ist.

8. System nach einem der Ansprüche 1 bis 7, wobei die Anordnung (1a) zwischen benachbarten Seiten der Platte befindet und sich darunter erstreckt, so dass die Anordnung in einem Hohlraum empfangbar ist, die in mindestens einem von dem ersten (01) und zweiten (02) Knochenabschnitt ausgebildet ist.

9. System nach einem der Ansprüche 1 bis 8, wobei sich die Anordnung im Verhältnis zu der unteren Oberfläche nach unten erstreckt und folglich unter einer Aufnahme befindet, die in der Platte (1) ausgebildet ist, wobei die Aufnahme von Seitenwänden begrenzt ist, die sich durch die obere und untere Oberfläche der Platte erstrecken, wobei die Seitenwände bemessen sind, um ein Einsetzen des dritten Befestigungselements (2) durch die Aufnahme und in das geneigte Loch (1a1) zu erlauben.

10. System nach Anspruch 9, wobei das geneigte Loch (1a1) einen ersten Durchmesser hat und ein Kopf des dritten Befestigungselements (2) einen zweiten Durchmesser hat, wobei der zweite Durchmesser größer als der erste Durchmesser ist.

11. System nach einem der Ansprüche 1 bis 10, das ferner Schrauben umfasst, die dazu bestimmt sind, in die Vielzahl von Löchern des Implantats eingesetzt zu sein.

## Claims

1. A system including an implant having a structure in the form of a plate (1) for compressing together first (O1) and second bone (02) parts separated by a joint, the implant comprising:
the plate (1) having a top surface, a bottom bone-contacting surface, and a plurality of holes (1b) formed through the top and bottom surfaces, wherein a first hole of the plurality of holes (1b) can be positioned on a first side of the joint, and a second hole of the plurality of holes (1b) can be positioned on a second side of the joint, the first and second holes being adapted to receive first and second fixation members (3), respectively, a central axis of the first hole extending into the first bone part (O1) but not the second bone part (02), and a central axis of the second hole extending into the second bone part (02) but not the first bone part (O1); and
a formation (1a) made of a rib forming an angled hole (1a1) extending through the plate (1) at an angle between 30° and 60° with respect to the bottom bone-contacting surface, the angled hole (1a1) being formed on a location on a determined portion of a length of the plate (1) and adapted to receive a third fixation member (2), a central axis of the angled hole (1a1) being angled with respect to a longitudinal axis of the plate, such that when the third fixation member (2) is inserted through the angled hole (1a1), it is arranged to extend into the first bone part (O1), across the joint, and into the second bone part (02) and to compress together the first bone part (O1) and the second bone part (02) in a direction along the longitudinal axis of the plate,
**characterized in that** the system further comprises a template of the plate for use in determining the positioning of the formation (1a) against bone, wherein the template does not include any rib.

2. The system of claim 1, wherein the first and second holes are locking holes.

3. The system of claim 2, wherein the first and second holes include threading for engaging with the first and second fixation members (3).

4. The system of any one of claims 1 to 3, wherein the plate (1) is curved so as to adapt to the curvature of the first (O1) and second (02) bone parts, the curvature of the plate arranging at least two of the fixation members at an angle with respect to one another.

5. The system of any one of claims 1 to 4, wherein at least three of the plurality of holes (1b) are arranged according to the corners of a triangle.

6. The system of any one of claims 1 to 4, wherein at least four of the plurality of holes (1b) are arranged according to the corners of a quadrilateral.

7. The system of any one of claims 1 to 4, wherein the angled hole (1a1) is aligned with the first and second holes of the plurality of holes (1b).

8. The system of any one of claims 1 to 7, wherein the formation (1a) is situated between and extends below adjacent sides of the plate, such that the formation is receivable in a cavity formed in at least one of the first (O1) and second (02) bones.

9. The system of any one of claims 1 to 8, wherein the formation extends below the bottom surface and is consequently situated below a slot formed in the plate (1), the slot is bounded by side walls extending through the top and bottom surfaces of the plate, the side walls being dimensioned to allow insertion of the third fixation member (2) through the slot and into the angled hole (1a1).

10. The system of claim 9, wherein the angled hole (1a1) has a first diameter, and a head of the third fixation member (2) has a second diameter, the second diameter being larger than the first diameter.

11. The system of any one of claims 1 to 10, further comprising screws for insertion into the plurality of holes of the implant.
